(19) Europäisches Patentamt
European Patent Office
Office européen des brevets

(11) EP 0 912 593 B1

(12) **FASCICULE DE BREVET EUROPEEN**

(45) Date de publication et mention de la délivrance du brevet:
**26.09.2001 Bulletin 2001/39**

(21) Numéro de dépôt: **97930587.7**

(22) Date de dépôt: **25.06.1997**

(51) Int Cl.$^7$: **C07H 21/00**

(86) Numéro de dépôt international:
**PCT/FR97/01134**

(87) Numéro de publication internationale:
**WO 97/49718 (31.12.1997 Gazette 1997/57)**

(54) **CONJUGUES D'UN OLIGONUCLEOTIDE/POLYMERE CONDUCTEUR ELECTRONIQUE AVEC UNE MOLECULE D'INTERET, ET LEURS UTILISATIONS**

KONJUGATE AUS EINEM OLIGONUKLEOTID UND EINEM ELEKTRONISCH LEITFÄHIGEM POLYMER MIT EINEM MOLEKÜL VON INTERESSE UND DEREN VERWENDUNG

CONJUGATES OF AN OLIGONUCLEOTIDE/ELECTRONIC CONDUCTOR POLYMER WITH A MOLECULE OF INTEREST, AND THEIR USES

(84) Etats contractants désignés:
**AT BE CH DE DK ES FI FR GB GR IE IT LI LU NL PT SE**

(30) Priorité: **25.06.1996 FR 9607846**

(43) Date de publication de la demande:
**06.05.1999 Bulletin 1999/18**

(73) Titulaire: **CIS BIO INTERNATIONAL**
**91400 Saclay (FR)**

(72) Inventeurs:
- **MARCHAND, Joseph**
  **F-91400 Orsay (FR)**
- **BAZIN, Hervé , Résidence "Les Genêts"**
  **F-30400 Villeneuve lès Avignon (FR)**

(74) Mandataire: **Vialle-Presles, Marie José et al**
**Cabinet Orès,**
**6, Avenue de Messine**
**75008 Paris (FR)**

(56) Documents cités:
**WO-A-94/22889**
**WO-A-95/29199**
**US-A- 5 391 723**

- **GARNIER F. ET AL: "Enzyme recognition by Polypyrrole Functionalised with Bioactive Peptides" JOURNAL OF THE AMERICAN CHEMICAL SOCIETY, vol. 116, no. 19, 1994, DC US, pages 8813-8814, XP002030299 cité dans la demande**
- **WOLOWACZ S.E. ET AL: "Covalent Electropolymerization of Glucose Oxidase in Polypyrrole" ANALYTICAL CHEMISTRY., vol. 64, no. 14, 1992, COLUMBUS US, pages 1541-1545, XP002030300 cité dans la demande**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention est relative à de nouvelles méthodes et de nouveaux composés permettant de contrôler la fixation de différentes molécules d'intérêt sur un polymère conducteur électronique (PCE) ;
La demande PCT WO 94/22889 au nom de CIS BIO INTERNATIONAL (Inventeurs TEOULE et al.), décrit la fixation d'oligonucléotides sur un support constitué d'un polymère conducteur électronique (PCE).
Cette fixation peut s'effectuer de différentes manières :

1) Par fixation d'un oligonucléotide présynthétisé sur le PCE (soit par réaction chimique de l'oligonucléotide sur le PCE préalablement fonctionnalisé, soit par copolymérisation des monomères de PCE avec le produit de condensation de l'oligonucléotide sur un desdits monomères).
2) Par élongation de l'oligonucléotide à partir d'un nucléoside, d'un nucléotide, ou d'un oligonucléotide déjà fixé au PCE, en utilisant par exemple une des méthodes classiques de synthèse des acides nucléiques.

La fixation d'oligonucléotides sur des PCE permet de faciliter l'obtention et l'utilisation de matrices d'oligonucléotides, qui sont en particulier utilisables pour le séquençage des acides nucléiques et le diagnostic.
De même que les matrices d'oligonucléotides, les matrices de peptides, et de manière plus générale, des matrices de diverses molécules constituent en effet un outil particulièrement intéressant, par exemple dans le domaine du diagnostic, ou pour le criblage de molécules actives. Il serait donc souhaitable que d'autres types de matrices puissent bénéficier des améliorations apportées par l'utilisation de PCE.
Toutefois, la fixation sur un PCE de molécules d'intérêt autres que les oligonucléotides, par exemple la fixation de peptides, pose plus de problèmes que la fixation d'oligonucléotides.
En effet, bien que des méthodes de synthèse de pyrrole portant un aminoacide ou un dipeptide soient décrites dans la littérature [GARNIER et al. J. Am. Chem. Soc., 116, 8813-8814 (1994)], il s'agit de méthodes de synthèse en solution qui, en pratique, ne permettent pas l'obtention, avec un rendement suffisant, de molécules peptidiques synthétiques de taille supérieure à 2 ou 3 acides aminés. Or, les molécules présentant un intérêt réel dans le domaine du diagnostic ou celui du criblage de molécules actives sont plus longues ; par exemple, un motif antigénique « minimal » comporte habituellement une moyenne de 6 aminoacides.
Les méthodes habituelles de synthèse peptidique sur support solide, dérivées de la technique de MERRIFIELD, font intervenir différents groupes protecteurs des chaînes latérales des aminoacides. La coupure de ces groupes, ainsi que la séparation du peptide et du support à l'issue de la synthèse sont effectuées en milieu acide fort (acide fluorhydrique ou acide trifluoracétique). Or, en milieu acide, les monomères de PCE, et en particulier le résidu pyrrole, ont tendance à polymériser, créant ainsi des sous-produits indésirables.
Il apparaît donc que ni la méthode de synthèse en solution, dont les possibilités sont limitées à la synthèse de di- ou de tripeptides, ni la synthèse sur support, dont la mise en oeuvre nécessite des conditions chimiques incompatibles avec la stabilité du résidu pyrrole, ne conviennent à la synthèse de peptides modifiés par un résidu pyrrole.
Il est toutefois possible de greffer un monomère de PCE, par exemple un résidu pyrrole, sur un oligopeptide préformé issu d'une synthèse peptidique traditionnelle sur phase solide. Ce greffage peut se faire par des méthodes connues, par réaction entre un dérivé carboxylique du pyrrole (activé par un réactif de couplage) et une des fonctions amines disponibles d'un peptide (par exemple la fonction amine de l'extrémité N-terminale) suivant le schéma ci-dessous [S.E. WOLOWACZ et al., Anal. Chem., 64, 1541-1545, (1992)].

$$\text{Pyrrole—COOR} + \text{NH2—peptide} \rightarrow \text{Pyrrole—CONH—peptide}$$

A l'issue de la réaction, il faut isoler le conjugué Pyrrole-peptide du mélange réactionnel contenant le peptide et le pyrrole n'ayant pas réagi, ainsi que les sels et sous-produits éventuels.
Les différentes méthodes dont on peut *a priori* envisager l'utilisation dans ce but, sont celles habituellement utilisées pour la purification des peptides, et en particulier les méthodes de chromatographie en phase inverse (RP-HPLC), ou de filtration sur gel.
La manière la plus simple de procéder à la détection des peptides à l'issue de la chromatographie, est de mesurer l'absorption dans l'ultraviolet, à une longueur d'onde de 215 nm à 220 nm; en effet, à cette longueur d'onde tous les peptides absorbent la lumière. Cependant, cette mesure de l'absorption vers 215 nm présente l'inconvénient d'être peu sensible, et de ne pas être spécifique, car des solvants ainsi que des ions organiques ou inorganiques sont également détectés.
Il existe des méthodes permettant de détecter spécifiquement les peptides, par une réaction chimique « en ligne» à la sortie de la colonne de chromatographie. Cette méthode présente l'avantage d'augmenter la sensibilité de la détection, mais en alourdit la mise en oeuvre, et en outre modifie irréversiblement le peptide ; cette modification

peut en particulier avoir des conséquences importantes sur ces propriétés fonctionnelles (par exemple son antigénicité).

Les problèmes exposés ci-dessus dans le cas des peptides se posent également pour la détection d'autres molécules d'intérêt etc... En effet, de nombreuses substances, telles que les sucres, les polyosides, les stéroïdes, ou bien ne présentent pas d'absorption spécifique à une longueur d'onde déterminée en UV, ou bien ne présentent qu'une absorption faible, ce qui nuit à la sensibilité de la détection.

On est alors amené à détecter, par un moyen physique ou chimique approprié, la présence de la molécule recherchée dans chaque fraction issue de la chromatographie, ce qui bien évidemment prend beaucoup de temps ; en outre, cette analyse est souvent destructive de l'analyte concerné.

La présente Invention a pour but l'obtention de conjugués, faciles à synthétiser et à purifier, de molécules d'intérêt avec un monomère de PCE. Dans ce but, les Inventeurs ont préparé des conjugués possédant des propriétés que ni la molécule d'intérêt, ni le monomère de PCE ne possèdent naturellement.

Dans ces conjugués, la molécule d'intérêt et le monomère de PCE sont reliés par l'intermédiaire d'une chaîne oligonucléotidique, faisant office de bras espaceur entre le monomère de PCE et la molécule d'intérêt considérée.

La présente invention a pour objet une macromolécule de formule (I) suivante :

$$\mathbf{P\text{-}O\text{-}M} \qquad (I)$$

dans laquelle :

M représente une molécule d'intérêt ;
O représente une chaîne oligonucléotidique ;
P représente un monomère d'un polymère conducteur électronique.

Au sens de la présente invention on entend par «molécule d'intérêt », toute molécule présentant une fonctionnalité utile dans des réactions sur support solide, par exemple des réactions de synthèse, ou de détection directe ou indirecte. Cette molécule d'intérêt peut être, par exemple, sans que cette liste soit limitative, une biomolécule, telle qu'une protéine (en particulier une enzyme) un acide aminé, un peptide, un glycopeptide, un lipide, un stéroïde, un glycolipide, un sucre, un polysaccharide, une molécule capable de générer, directement ou indirectement, un signal, ou bien une molécule complexe, multifonctionnelle; etc. Avantageusement, cette molécule d'intérêt constitue l'un des membres d'un couple d'affinité, il peut s'agir par exemple de la biotine, ou d'un peptide potentiellement antigénique, etc.

P peut par exemple être un monomère de polyacétylène, de polyazine, de poly(p-phénylène), de poly(p-phénylène vinylène), de polypyrène, de polypyrrole, de polythiophène, de polyfuranne, de polysélénophène, de polypyridazine, de polycarbazole, de polyaniline, etc.

Avantageusement, P est un groupe pyrrole.

La chaîne oligonucléotidique O peut être constituée par l'assemblage de nucléotides naturels et/ou de d'analogues de nucléotides, tels que ceux décrits par exemple par UHLMANN, [Chemical Review, 90:4, 543-584 (1990)]. Il peut s'agir d'un oligonucléotide simple-brin, ou d'un oligonucléotide double-brin sur au moins une partie de sa longueur. Dans le deuxième cas, l'un des brins est fixé de manière covalente au monomère P, et l'autre brin est fixé de manière covalente à la molécule d'intérêt M.

De façon théorique il n'y a pas de limitation dans la nature ou la longueur de l'oligonucléotide; en pratique, la chaîne oligonucléotidique aura avantageusement une longueur comprise entre 6 et 60, préférentiellement entre 10 et 30 nucléotides. Avantageusement le pourcentage en (G+C) de l'oligonucléotide O est inférieur ou égal à 70%, et de préférence inférieur ou égal à 50%.

La fixation de l'oligonucléotide O sur le monomère de PCE peut être effectuée comme décrit dans la demande PCT WO 94/22889. La fixation de la molécule d'intérêt M sur l'oligonucléotide O peut être effectuée par différents procédés, connus en eux-même, permettant de lier un oligonucléotide à une autre molécule. Le choix du procédé le plus approprié dépend essentiellement de la nature de la molécule d'intérêt M.

Par exemple, l'oligonucléotide O peut être activé par fixation d'un ester de N-hydroxy-succinimide d'acide aminé, d'un groupe SH [ARAR et al., Bioconjugate Chem. 6, p. 573-577, (1995)], ou d'un groupe maléimide.

La présente invention a également pour objet un procédé de préparation d'une macromolécule P-O-M telle que définie ci-dessus, à partir d'un mélange comprenant ladite macromolécule ainsi que les réactifs P-O et M à partir desquels elle a été formée, lequel procédé est caractérisé en ce qu'il comprend au moins une étape au cours de laquelle on procède au fractionnement dudit mélange, par tous moyens permettant de séparer les fractions comprenant respectivement M et P-O-M, de la fraction comprenant P-O, et une étape au cours de laquelle l'on procède à la détection de la fraction comprenant P-O-M, et/ou à la détermination de la quantité de P-O-M par détection et/ou mesure d'un paramètre associé à l'oligonucléotide O et non-associé à la molécule d'intérêt M.

La chaîne oligonucléotidique O joue, par sa nature chimique et par sa longueur, un rôle dans les propriétés physiques (polarité en chromatographie, par exemple) du conjugué avec la molécule d'intérêt considérée, ce qui permet d'ajuster une séparation optimale du conjugué obtenu et des réactifs en excès. D'autre part, elle permet la détection spécifique du conjugué dans l'ultraviolet, à une longueur d'onde comprise entre 240 et 270 nm (avantageusement, 265 nm), et éventuellement par hybridation avec un oligonucléotide de séquence complémentaire.

Les propriétés conférées par la chaîne oligonucléotidique O permettent ainsi une manipulation aisée de quantités infimes de molécules d'intérêt, en facilitant leur séparation par des méthodes d'ultrafiltration, et/ou de chromatographie de partition, ou de chromatographie d'affinité sur des oligonucléotides de séquence complémentaire, ainsi que leur détection et leur quantification rapide par une mesure d'absorption spécifique entre 240 et 270 nm. Ceci est particulièrement avantageux lorsque la molécule d'intérêt M est un peptide ; en effet, la mesure de l'absorption des oligonucléotides à 265 nm est beaucoup plus sensible et spécifique (elle n'est pas perturbée par les sels et les solvants) que celle de l'absorption des peptides à 205 nm.

La présente invention a pour également pour objet des copolymères dont au moins l'une des unités est constituée par une macromolécule P-O-M telle que définie ci-dessus.

Des polymères conformes à l'invention sont par exemple définis par l'une des formules (II), (III) ou (IV) suivantes :

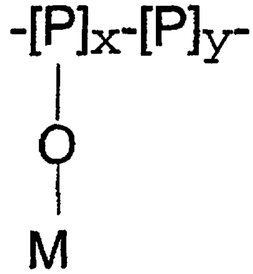

(II)

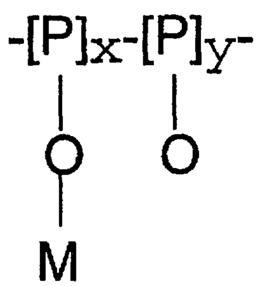

(III)

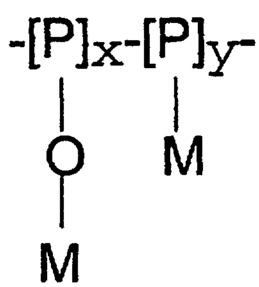

(IV)

dans lesquelles :

x et y représentent des nombres entiers égaux ou supérieurs à 1, et
M représente une molécule d'intérêt ;
O représente une chaîne oligonucléotidique
P représente un monomère d'un polymère conducteur électronique.

Dans un copolymère conforme à l'invention, les monomères P de polymère conducteur électronique, peuvent être identiques entre eux, ou bien de nature différente ; les oligonucléotides O peuvent également être identiques entre eux, ou différents par leur séquence et/ou par leur taille, et/ou leur nature simple- ou double-brin ; de même, les molécules M peuvent être identiques ou différentes entre elles.

Ces copolymères peuvent être préparés par copolymérisation d'un ou plusieurs conjugué(s) P-O-M purifié (s), ou tel que défini(s) ci-dessus, avec des monomères P, et/ou avec des conjugués P-O, tels que ceux décrits dans la demande PCT WO 94/22889, et/ou avec des conjugués P-M; ils peuvent également être obtenus par fixation d'une ou plusieurs molécules d'intérêt M sur tout ou partie des chaînes oligonucléotidiques latérales d'un copolymère PCE/ oligonucléotide, tel que ceux décrits dans la demande PCT WO 94/22889. Cette fixation peut par exemple être effectuée par liaison covalente d'une molécule d'intérêt M avec un oligonucléotide O, lui-même fixé au PCE, ou bien par l'intermédiaire d'un oligonucléotide hybridé avec ledit oligonucléotide O sur au moins une partie de sa longueur.

Les copolymères conformes à l'invention peuvent être utilisés dans toutes les applications dans lesquelles il est habituel de fixer des molécules d'intérêt sur un support solide, et en particulier sur une électrode.

La présente invention a pour objet des électrodes dont la surface porte au moins un copolymère conforme à l'Invention.

Par exemple, la surface d'une électrode conforme à l'Invention peut être entièrement recouverte d'un seul copolymère conforme à l'Invention ; elle peut également porter plusieurs copolymères conformes à l'invention, et différant entre eux par au moins un des constituants P, O, ou M,; ce ou ces copolymères peuvent également être associés, sur la même électrode, à d'autres polymères, par exemple des copolymères PCE/oligonucléotides, tels que ceux décrits dans la demande PCT WO 94/22889, ou bien à des polymères de nature différente, tels que par exemple des PCE résultant de la polymérisation de monomères P tels que définis ci-dessus.

Des polymères conformes à l'Invention sont utilisables pour la constitution de matrices de molécules d'intérêt, en particulier de matrices d'électrodes comprenant au moins une électrode conforme à l'invention, telle que définie ci-dessus.

Des électrodes constituant des points différents d'une même matrice peuvent différer entre elles par les monomères P entrant dans la composition des copolymères présents à leur surface, et/ou par les oligonucléotides O et/ ou les molécules M des chaînes latérales de ces copolymères; elles peuvent également différer entre elles par la quantité de ces chaînes latérales par unité de surface.

Certaines des électrodes de la même matrice peuvent porter un polymère autre qu'un copolymère conforme à l'invention, par exemple, un copolymère PCE/oligonucléotide tels que ceux décrits dans la demande PCT WO 94/22889. Selon l'utilisation envisagée, ce copolymère PCE/oligonucléotide pourra être conservé tel quel, ou bien être utilisé pour la fixation d'autres molécules d'intérêt, directement, ou par hybridation avec un autre oligonucléotide portant la molécule d'intérêt.

De telles matrices peuvent être préparées en procédant au dépôt adressé des polymères souhaités sur des électrodes déterminées ; par exemple des copolymères conformes à l'invention, peuvent être déposés par copolymérisation électrochimique adressée, sur les électrodes choisies, de conjugués P-O-M, et/ou de quantités variables d'un même conjugué P-O-M, avec les monomères P, et éventuellement des conjugués P-O, et P-M. L'utilisation des macromolécules P-O-M et des copolymères conformes à l'invention permet donc l'obtention facile de matrices multifonctionnelles.

Les conjugués P-O-M conformes à l'invention peuvent également être utilisés pour étalonner des matrices d'oligonucléotides fixés sur un support de polymère conducteur électronique. Les Inventeurs ont en effet constaté que l'on pouvait établir une corrélation reproductible entre la quantité de chaînes latérales O-M, et la quantité d'oligonucléotides fixés sur un support de polymère conducteur électronique. En conséquence, la mesure de la fixation des chaînes latérales O-M sur une électrode, dans des conditions expérimentales données, permet de prévoir la quantité d'oligonucléotides qui sera fixée sur une autre électrode (de la même matrice ou d'une autre matrice), dans les mêmes conditions expérimentales.

L'utilisation des conjugués P-O-M et des copolymères conformes à l'invention permet d'obtenir des électrodes constituant des témoins internes, et permettant un contrôle qualitatif et/ou quantitatif de la fixation de molécules X à un support solide constitué par la surface d'une électrode portant un polymère conducteur électronique. Ce contrôle de la fixation des molécules X peut être effectué à tout moment (après le dépôt de celles-ci ou en cours d'utilisation). Les molécules X dont la fixation peut être détectée en utilisant un copolymère conforme à l'Invention peuvent être de nature variée ; avantageusement, elles comprennent au moins une chaîne oligonucléotidique O et/ou une molécule d'intérêt M qui peuvent être identiques à ou différentes de celles du copolymère conforme à l'invention utilisé.

Un polymère conforme à l'invention peut être utilisé pour détecter et/ou quantifier une molécule X qui constitue l'une des chaînes latérales du même polymère, ou bien d'un polymère de même formule, déposé sur la même électrode, ou bien sur une autre électrode de la même matrice ou d'une matrice différente. Il peut également être utilisé pour détecter et/ou quantifier une molécule X appartenant à un polymère différent, qui peut également être déposé sur la même électrode, sur une autre électrode de la même matrice ou d'une matrice différente.

Cette détection et/ou quantification peut être effectuée par exemple en détectant les chaînes oligonucléotidiques O des copolymères conformes à l'invention par hybridation avec une sonde marquée, ou bien en utilisant sur une ou plusieurs des électrodes de la matrice, des copolymères conformes à l'invention comprenant une molécule d'intérêt M constituant un marqueur facile à détecter (par exemple la biotine, ou un marqueur fluorescent).

La présente invention sera mieux comprise à l'aide du complément de description qui va suivre, qui se réfère à des exemples de préparation et d'utilisation de macromolécules et de copolymères conformes à l'invention.

**EXEMPLE 1: PROPRIETES SPECTRALES ET CHROMATOGRAPHIQUES DE PEPTIDES**

Pour illustrer les problèmes posés par la détection d'un peptide d'intérêt biologique, on utilise un peptide synthétique commercial (réf. A2532 SIGMA-ALDRICH Chimie), de masse molaire 1652,1 Da, correspondant au fragment 11-24 de l'ACTH (adrenocortotrophic hormone). D'après les spécifications du fabricant, la préparation contient 61% de peptide.

On injecte sur une colonne HPLC LICHROSPHER® RP-18E 5 µm 125-4 MERCK, DARMSAT,DE) 25 µl d'une solution à 2mg/ml dudit peptide. L'élution (1ml/mn) est effectuée par le mélange A + B, (A = 5% ACN (acétonitrile) 25 mM TEAAc (acétate de triéthyl ammonium) ; B = 50% ACN 25 mM TEAAc) en utilisant un gradient de 0% à 100% B en 35 minutes.

On observe un pic à un temps de rétention Rt = 2 minutes et un pic assez large à Rt = 10 minutes. Les fractions correspondant à ces deux pics sont collectées, concentrées et analysées.

La fraction F1 (Rt = 2 minutes) montre une absorption $A_{220\ nm}$ = 1,033. D'autre part, $A_{275\ nm}$ est du même ordre que le bruit de fond (0,005). Cette fraction contient donc des sels ($A_{220\ nm}$ non spécifique).

La fraction F2 (Rt = 10 minutes) présente une absorption $A_{220nm}$ = 1,90 et $A_{275\ nm}$ = 0,073. Cette fraction contient donc le peptide.

Ceci illustre le fait que la détection U.V. vers 215 à 220 nm ne permet pas, à elle seule, de différencier sur le chromatogramme le signal dû au peptide des signaux parasites d'autres substances.

On a analysé de la même façon par RP-HPLC le peptide synthétique commercial (réf. A 0673 SIGMA-ALDRICH CHIMIE), de masse molaire 2465,7 Da, correspondant au fragment ACTH (18-39). D'après le fabricant, l'échantillon contient 82% de peptide.

L'analyse HPLC est réalisée dans les mêmes conditions que ci-dessus, et les fractions sont détectées en U. V. à 215 nm.

On observe un pic majeur pour Rt = 18,45 minutes ; ce pic est plus fin que celui observé pour le fragment ACTH (11-24).

Cette expérience montre que deux fragments peptidiques différents présentent des pics caractérisés par des temps de rétention très différents. La comparaison des profils HPLC montre également que les pics, comme dans le cas de ACTH (11-24), peuvent être élargis.

**EXEMPLE 2 : SYNTHESE D'UN OLIGONUCLEOTIDE DOUBLEMENT MODIFIE PAR UN RESIDU PYRROLE ET UN BRAS AMINOALKYLE.**

Un oligonucléotide modifié de séquence :

$$5'HO\text{-}dC^{pyrrole}\text{-}(T)_{10}\text{-}p\text{-}dC^{aminohexyl}\text{-}p\text{-}dTOH3'$$

est synthétisé sur support solide (CPG (Controled pore Glass)) par la méthode dénommée « phosphite-phosphoramidite », décrite par BEAUCAGE et LYER, [Tetrahedron., 48, 2223-2311, (1992)].

Les principales étapes de cette synthèse sont indiquées ci-dessous.

On prépare un aminoalkyle-phosphoramidite dérivé de la 5'-O-(4,4'-diméthoxytrityl)-N-4-(6-aminohexyl)-2'-désoxycytidine [ROGET et al. Nucleic Acids Res., 17, 7643-7650, (1989)].

Cet aminoalkyle-phosphoramidite est obtenu en deux étapes : l'amine primaire du bras alkylamine porté par le nucléoside est protégée par un groupe trifluoracétyle, suivie d'une phosphitylation de l'hydroxyle en 3' par un procédé analogue à celui décrit par SPROAT et al.[Nucleic Acids Res. 15, 6181-6196, (1987)], pour le phosphoramidite de la 5'-trifluoracetamido-2',5'-didésoxythymidine par SPROAT et al.[Nucleic Acids Res. 15, 6181-6196, (1987)].

On couple les aminoalkyle-phosphoramidites ainsi obtenus sur une colonne d'un synthétiseur d'ADN, par l'intermédiaire de thymidines préalablement fixées sur cette colonne.

D'autres aminoalkyle-phosphoramidites tels que ceux décrits par AGRAWAL et al., [Nucleic Acids. Res., 14, 6227, (1986)] ; CONNOLLY, [Nucleic Acids. Res. 15, 3131, (1987)] ; BEAUCAGE et LYER, [Tetrahedron. 49, 1925-1963, (1993)] sont également utilisables.

On condense ensuite le phosphoramidite de la thymidine sur l'aminoalkyle-phosphoramidite fixé à la colonne. Cette étape est répétée jusqu'à obtention d'une chaîne oligonucléotidique de 10-mères.

On condense, à l'extrémité 5' de l'oligonucléotide ainsi obtenu, un phosphoramidite de la 5'-O-(4,4'-diméthoxytrityl)-N-4-(6-aminohexyl-)-2'-désoxycytidine sur lequel a été greffé un résidu pyrrole, selon le protocole décrit dans la demande PCT WO 94/22889 et par LIVACHE et al., [Nucleic Acids Res. 22, 2915-2921, (1994)].

L'oligonucléotide est déprotégé dans l'ammoniaque concentrée (16 heures à 55°C), et purifié par HPLC sur une colonne LICHROSPHER® RP-18E 250-10 (10 µm) (MERCK, DARMSTAT, DE) par un gradient d'acétonitrile dans l'acétate de triéthylammonium 50 mM (tampon A : 5% acétonitrile, tampon B : 50% acétonitrile ; débit 5 ml/minute, gradient de 10% B à 25% B en 20 minutes), selon la méthode décrite dans : « Oligonucleotide synthesis : A practical approach. Ed M.J. Gait. IRL Press, Oxford ».

Les fractions correspondant à un pic majoritaire (temps de rétention supérieur à 15 minutes) sont évaporées. Après évaporation, l'oligonucléotide obtenu est dessalé par filtration sur une colonne NAP-5® (PHARMACIA-LKB BIOTECHNOLOGY, UPPSALA, Suède).

**EXEMPLE 3 : PREPARATION D'UN OLIGONUCLEOTIDE ACTIVE SOUS LA FORME D'ESTER DE N-HYDROXY SUCCINIMIDE.**

L'oligonucléotide modifié (dénommé ci-après pyr-$T_{10}$-NH2) préparé selon la méthode décrite à l'exemple 2 ci-dessus, est transformé en son intermédiaire activé (ester de N-hydroxysuccinimide) correspondant selon le protocole suivant :

L'oligonucléotide pyr-$T_{10}$-NH2 (10 à 25 nmol) lyophilisé est repris dans 12 µl de tampon 50 mM N-(3-sulfopropyl)morpholine (MOPS) pH 7,0. A cette solution sont ajoutés 28 µl de diméthylformamide contenant 4 µmol de dissuccinimidyl subérate (DSS PIERCE ROCKFORD, IL), et le mélange est laissé sous agitation mécanique (16 heures à 4°C ou 5 heures à 20°C). On dépose le mélange réactionnel sur une colonne NAP-5® équilibrée avec de l'eau, et la colonne est éluée par de l'eau selon le protocole préconisé par le fabricant. La fraction exclue (1 ml) est extraite 5 fois par 1 ml de n-butanol. A chaque extraction, on centrifuge, on élimine la phase supérieure (organique) et on conserve la phase inférieure (aqueuse). A la dernière extraction, l'ester de N-hydroxy succinimide (dénommé ci-après pyr-$T_{10}$-NHS) précipité au fond du tube est séché sous vide (SPEED-VAC) puis conservé à -20°C (préférablement moins de 24 heures) jusqu'à son utilisation.

Une HPLC analytique d'une partie aliquote du mélange réactionnel à différents temps de réaction est réalisée sur une colonne LICHROSPHER® RP-18E/125-4 (5 µm)(MERCK, DARMSTAT, DE) ; on effectue l'élution par un gradient d'acétonitrile dans l'acétate de triéthylammonium 50 mM (tampon A : 5% acétonitrile, tampon B : 50% acétonitrile); débit 1 ml/mn, gradient de 10% B à 30% B en 20 minutes, puis de 30% B à 50% B en 15 minutes. Cette analyse chromatographique montre la disparition du pic de l'oligonucléotide pyr-$T_{10}$-$NH_2$, (temps de rétention environ 19 minutes) et l'apparition d'un pic (temps de rétention environ 27 minutes) correspondant à l'ester de N-hydroxy succinimide (dénommé pyr-$T_{10}$-NHS).

**EXEMPLE 4 : COUPLAGE D'UN OLIGONUCLEOTIDE MODIFIE SUR DES PEPTIDES**

**A) Couplage sur le fragment ACTH(11-24) :**

20 nmol de pyr-$T_{10}$-NHS obtenu comme décrit dans l'exemple 3, sont repris dans 50 ml de tampon MOPS (50 mM, pH 7,8). On ajoute le peptide ACTH(11-24) (SIGMA, St Louis, Etats-Unis) (26 nmol, 1,3 eq.) dans 50 µl de tampon MOPS pH 7,8. On incube une nuit à 4°C.

On observe en HPLC analytique dans des conditions identiques à celles de l'exemple 3 la disparition de pyr-$T_{10}$-NHS, et l'apparition d'un pic majoritaire correspondant au conjugué oligonucléotide-peptide (temps de rétention environ 26,4 minutes.

Le mélange réactionnel est purifié par HPLC-semi-préparatoire sur une colonne LICHROSPHER® RP-18E/125-4 (5 µm), par un gradient d'acétonitrile dans l'acétate de triéthylammonium 50 mM. L'élution est effectuée dans les mêmes conditions que la HPLC analytique décrite à l'exemple 3.

On détecte par mesure UV à 265 nm le conjugué oligonucléotide-peptide et on le collecte dans la fraction correspondant à un temps de rétention compris entre 24 et 25,5 minutes ; cette fraction est séchée par évaporation (SPEED-VAC). On obtient ainsi environ 3,7 nmol de conjugué dénommé pyr-$T_{10}$-ACTH(11-24).

**B) Couplage sur le fragment ACTH(18-39) :**

L'oligonucléotide-ester pyr-$T_{10}$-NHS (EX. 3) (environ 20 nmol) est couplé avec environ 35 nmol soit environ 1,8 eq. du fragment ACTH(18-39) (SIGMA, St Louis, Etats-Unis), et le produit de couplage, dénommé pyr-$T_{10}$-ACTH

(18-39), est purifié en suivant les protocoles décrits en A) ci-dessus.

Le conjugué pyr-$T_{10}$-ACTH(18-39) est détecté dans la fraction correspondant à un temps de rétention compris entre 26 à 27 minutes. Après séchage de cette fraction, on obtient environ 7 nmol du conjugué.

**EXEMPLE 5 : SYNTHESE D'UN CONJUGUE PYRROLE-$T_{10}$-BIOTINE :**

Un oligonucléotide modifié de séquence pyr-$T_{10}$-NH2 (10 à 20 µmol) est traité par un excès de biotine-NHS (environ 50 équivalents) (SIGMA) dissous dans 20 µl de diméthyl-formamide dans un tampon carbonate (1 M) à pH 9 ; on incube pendant 30 minutes à 20°C.

Le mélange réactionnel est purifié sur une colonne d'exclusion (NAP PHARMACIA) et le conjugué pyr-$T_{10}$-bio (Rt = 23 minutes) est séparé de l'oligonucléotide pyr-$T_{10}$-NH2 résiduel (Rt = 18 minutes) par HPLC analytiques dans les conditions de l'exemple 3. Les fractions sont détectées à 265 nm.

**EXEMPLE 6 : ELECTROPOLYMERISATION DES CONJUGUES PYR-$T_{10}$-PEPTIDE, ET PYR-$T_{10}$-BIOTINE.**

Les conjugués synthétisés comme décrit dans les exemples 4 A), 4 B) et 5, sont déposés, par électropolymérisation sur des micro-électrodes de silicium recouvertes d'or.

Ces micro-électrodes sont disposées de façon à former une matrice en forme de « damier » ; cette matrice est formée de micro-électrodes carrées de 50 µm de côté disposées en 5 colonnes et 4 lignes selon le schéma suivant (n = 5 colonnes et p = 4 lignes). Les micro-électrodes sont repérées par leur coordonnées (i;j).

L'électropolymérisation est réalisée en plongeant la matrice d'électrodes dans un milieu contenant le conjugué pyrrole-oligonucléotide-biomolécule concerné, et du pyrrole (rapport molaire Pyrrole/conjugué = 10 000 environ), dans une solution $LiClO_4$ 0,1M. On relie l'électrode sur laquelle on souhaite effectuer le dépôt à un potentiostat, et on effectue des cycles entre -0,35 V et +0,85 V (potentiels mesurés par rapport à une électrode au calomel reliée à la cellule d'électrolyse et connectée au potentiostat) à une vitesse de 100 mV/s. La contre-électrode est constituée d'un fil de platine.

Certaines des micro-électrodes sont recouvertes d'un copolymère formé de polypyrrole et d'un conjugué :

- pyr-$T_{10}$-ACTH (18-39): électrodes (1;4) et (3;4)
- pyr-$T_{10}$-bio : électrodes(2;3) et (4;4).

Les électrodes restantes sont ou bien laissées dans leur état initial, c'est-à-dire que le dépôt d'or reste inchangé (OR), ou bien recouvertes de polypyrrole non modifié (PP) ; ces électrodes constituent des témoins négatifs permettant d'évaluer la spécificité des réactions effectuées sur la matrice.

Le tableau I ci-dessous schématise la matrice de microélectrodes, et l'emplacement des différents dépôts.

TABLEAU I

| i \ j | 1 | 2 | 3 | 4 | 5 |
|---|---|---|---|---|---|
| 1 | OR | OR | OR | ACTH | OR |
| 2 | OR | OR | BIO | OR | OR |
| 3 | PP | PP | PP | ACTH | PP |
| 4 | PP | PP | PP | BIO | PP |

OR : pas de dépôt

PP : Dépôt de polypyrrole

**EXEMPLE 7 : MISE EN EVIDENCE DES BIOMOLECULES ELECTROPOLYMERISEES SUR LES ELECTRODES**

La matrice d'électrodes réalisée dans l'exemple 6 est incubée en présence d'un conjugué streptavidine-phycoérythrine (solution commerciale streptavidine-R-phycoérythrine MOLECULAR PROBES, à 1mg/ml diluée au 1/20 dans du tampon phosphate 10mM pH 7,4 contenant 0,5M NaCl et 0,05% de TWEEN 20).

L'observation sous un microscope à épifluorescence révèle que la fluorescence est localisée sur les électrodes (2;3) et (4;4). Il n'y a pas de fluorescence parasite sur les autres électrodes, ce qui montre que le conjugué pyrrole-oligonucléotide-biotine s'est fixé spécifiquement sur les électrodes cible souhaitées.

La même matrice de micro-électrodes est incubée en présence d'un anticorps biotinylé spécifique de la partie C-terminale du peptide 18-39 de l'ACTH (anticorps ACR-17-bio: CIS BIO INTERNATIONAL) puis en présence d'un conjugué streptavidine/phycoérythrine pour révéler le produit de la réaction.

L'observation sous microscope à épifluorescence révèle que seules les micro-électrodes portant le conjugué pyrrole-oligonucléotide-ACTH, et celles portant la biotine sont fluorescentes, ce qui montre que le conjugué pyrrole-oligonucléotide-ACTH s'est fixé spécifiquement sur les électrodes cible souhaitées, qu'il est accessible à un anticorps, et que ses propriétés antigéniques sont conservées.

**EXEMPLE 8 :**

En utilisant la méthode décrite dans l'Exemple 4, on couple le fragment ACTH(18-39) sur un oligonucléotide-pyrrole ester de N-hydroxysuccinimide, préparé par le procédé décrit dans l'exemple 3, à partir d'un oligonucléotide-pyrrole de séquence 5'pyr-$T_9$-NH2, synthétisé suivant le protocole décrit dans l'exemple 2.

On dispose ainsi des conjugués de type P-O-M :

I. pyr-$T_{10}$-ACTH(11-24)
II. pyr-$T_{10}$-ACTH(18-39)
III. pyr-$T_9$-ACTH(18-39)
IV. pyr-$T_{10}$-Biotine
V. pyr-$T_5$-HCVG (formé de l'association d'un bras $T_5$ et d'une séquence spécifique du génome du virus HCV).

En suivant le procédé d'électropolymérisation de l'exemple 6, on effectue un dépôt électrochimique des conjugués I à V sur un réseau de micro-électrodes carrées de 100 µm de côté.

Le tableau II ci-dessous schématise la matrice de microélectrodes, et l'emplacement des différents dépôts.

On laisse certaines électrodes non recouvertes (OR) ou recouvertes de polypyrrole non modifié (PP).

TABLEAU II

| i \ j | 1 | 2 | 3 | 4 | 5 | 6 | 7 | 8 |
|---|---|---|---|---|---|---|---|---|
| 1 | OR | IV | I | I | III | OR | V | PP |
| 2 | PP | III | II | II | IV | OR | V | OR |

On incube cette plaquette de microélectrodes avec une sonde oligonucléotidique de séquence $dC^{Biotine}(dA)_9$ préparée et purifiée suivant l'exemple 2, et en utilisant le phosphoramidite biotine décrit par ROGET et al. [Nucleic Acids Res., 17,7643-7650 (1989)].

L'hybridation est effectuée avec environ 8 pmoles de sonde oligonucléotidique dans 200 µl de tampon d'hybridation (tampon phosphate 0,1 M, pH 7,4 contenant 0,5 M Nacl/TWEEN, 30 mn à 20°C puis 30 mn à 4°C.

On révèle ensuite par incubation (10 mn à 4°C) dans une solution de streptavidine-phycoérythrine (solution commerciale streptavidine-R-phycoérythrine MOLECULAR PROBES à 1 mg/ml) diluée au 1/20 dans du tampon d'hybridation.

Après examen sous microscope à fluorescence, on observe que les électrodes (1;2) et (2;5) sont fortement positives, que les électrodes (1;3), (1;4), (2;3)et (2;4) sont positives, alors que les électrodes (1;5) et (2;2) montrent une fluorescence plus faible ; toutes les autres électrodes et, entre autres (1;7) et (2;7) ne présentent pas de fluorescence au-dessus du bruit de fond.

Le conjugué IV (pyr-$T_{10}$-Biotine) permet de servir de contrôle positif de révélation. Lors d'une première utilisation de la plaque, il permet de certifier la révélation par le complexe streptavidine/ phycoérythrine. Les 4 microélectrodes comportant le conjugué de type P-O-M dans lequel O est un oligonucléotide de séquence $T_{10}$. (1;3), (1;4) (2;

3) et (2;4) présentent une hybridation positive, ce qui permet de certifier que le composé de type P-O-M a été copolymérisé sur ces électrodes.

La faible hybridation observée sur les électrodes (1;5) et (2;2) et l'absence d'hybridation sur les électrodes (1;7) et (2;7) montre la sélectivité de l'hybridation et illustre la limite inférieure pour la taille de la partie oligonucléotidique du conjugué permettant cette dilution par hybridation, que l'on peut estimer entre 5 et 9 nucléotides de long.

**EXEMPLE 9 :**

On utilise le conjugué de type P-O-M Pyr-$T_{10}$-Bio décrit dans l'exemple 5, en solution dans l'eau à une concentration de 140 $UA_{265}$/ml, ce qui permet de déterminer la concentration molaire, soit 1,17 µmole/ml (avec $\varepsilon_{265}$=120000)grâce aux propriétés d'absorption dans l'UV de la partie O de ce conjugué.

On effectue une gamme séquentielle de dilutions du conjugué (1/5 ; 1/25 ; 1/50 ; 1/250 ; 1/1250) dans de l'eau et on ajoute 10 µl de chaque dilution à 300 µl de solution de pyrrole 20 mM dans $LiClO_4$ 0,1M.

A l'aide de solutions contenant des concentrations variables de conjugué Pyr-$T_{10}$-Bio on effectue des copolymérisations sur des microélectrodes comme décrit dans l'exemple 6. Le rapport molaire pyrrole/conjugué est différent pour chaque électrode.

Le tableau III ci-dessous schématise la matrice de microélectrodes, et l'emplacement des différents dépôts.

TABLEAU III

| j / i | 1 | 2 | 3 | 4 | 5 | 6 |
|---|---|---|---|---|---|---|
| 1 | OR | 1/5 | 1/25 | 1/50 | 1/250 | 1/1250 |
| 2 | PP | 1/5 | 1/25 | 1/50 | 1/250 | 1/1250 |

On effectue la révélation par incubation dans une solution au 1/20$^{ème}$ de streptavidine/phycoérythrine dans un tampon phosphate 10mM pH 7,4 contenant 0,5M NaCl et 0,05% de Tween 20.

On observe la plaque d'électrodes sous un microscope à épifluorescence couplé à une caméra CCD (HAMAMATSU), elle même connectée à un microordinateur pourvu d'un programme d'analyse d'image.

On peut fixer le temps d'exposition pour faire varier la sensibilité de la détection.

En utilisant des temps d'exposition de 0,5 s, 1 s, 2 s, 4 s, 8 s, 16 s, on observe qu'au moins les trois plots contigus (1;2), (1;3) (1;4) donnent toujours des signaux de fluorescence, qui sont d'intensité croissante (1;2 > (1;3) > 1;4). Les plots (1;1) et (2;1) permettent d'apprécier le bruit de fond).

Les signaux fournis par les plots (1;2) et (2;2), (1;3) et (2;3) etc... sont du même ordre deux à deux, ce qui permet d'apprécier la reproductibilité.

Ceci permet de montrer que les intensités de fluorescence sont corrélées avec les quantités initiales de composé de type P-O-M (quantifiées à l'origine par absorption à 265 nm) introduites dans les différentes solutions d'électrolyte.

**EXEMPLE 10 :**

a) Des quantités variables d'un conjugué pyrrole-oligonucléotide, (pyrrole-$T_{10}$-k-ras), de formule Pyr-5'($T_{10}$)-k-$ras_{(28-41)}$, dans lequel (28-41) représente la séquence des nucléotides 28 à 41 du brin sens de la séquence k-ras humaine portant une mutation G→A sur le nucléotide 34, ont été électropolymérisées, comme décrit dans l'exemple 6, en présence d'une quantité constante de pyrrole (600 µl de pyrrole 20mM dans $LiClO_4$, soit 1,2 × $10^{-5}$ mol), sur 5 microélectrodes différentes d'une plaquette de microélectrodes carrées (50 µm × 50 µm) recouvertes d'or, comme indiqué sur le tableau IV ci-après.

TABLEAU IV

| Dépôt | Quantité de conjugué | Rapport pyrrole/conjugué |
|---|---|---|
| 1 | $3 \times 10^{-9}$ mol | 4 000 |
| 2 | $6 \times 10^{-10}$ mol | 20 000 |
| 3 | $1,2 \times 10^{-10}$ mol | $10^5$ |
| 4 | $2,4 \times 10^{-11}$ mol | $5 \times 10^5$ |
| 5 | $4,3 \times 10^{-12}$ mol | $2,5 \times 10^6$ |

b) Sur 5 autres microélectrodes de la même plaquette, on procède de même avec le conjugué de type P-O-M pyrrole-$T_{10}$-biotine, en utilisant la gamme illustrée dans le tableau V ci-après, et sur la figure 1(b):

TABLEAU V

| Dépôt | Quantité de conjugué | Rapport pyrrole/conjugué |
|---|---|---|
| 1 | $6,6 \times 10^{-9}$ mol | 1800 |
| 2 | $6,6 \times 10^{-10}$ mol | 18000 |
| 3 | $1,3 \times 10^{-10}$ mol | $9 \times 10^4$ |
| 4 | $2,6 \times 10^{-11}$ mol | $4,5 \times 10^5$ |
| 5 | $5,3 \times 10^{-12}$ mol | $2,25 \times 10^6$ |

On incube la plaquette pendant 30 mn à 45°C, en présence de l'oligonucléotide complémentaire de la séquence k-ras$_{(28-41)}$, biotinylé en 5'. On utilise une solution à 2UA$_{260}$/ml d'oligonucléotide biotinylé, diluée au 1/1000ème dans du tampon PBS 10mM pH 7,4 contenant 0,5M NaCl et 10mM EDTA. Après lavage (20°C) par du tampon PBS 10mM contenant 0,5M NaCl et 0,05% de TWEEN 20, on incube la plaquette (10mn à 20°C) dans une solution de streptavidine-phycoérythrine (solution commerciale streptavidine-R-phycoérythrine MOLECULAR PROBES 1 mg/ml) diluée au 1/20 dans du tampon PBS 10mM, pH 7,4 contenant 0,5M de NaCl et 0,05% de TWEEN 20.

On observe la plaquette sous un microscope à épifluorescence (OLYMPUS) équipé d'une caméra CCD (temps d'intégration $t_i$=2s ; gain = $\times$ 4) relié à un ordinateur équipé d'un logiciel d'analyse d'image (IMAGE PRO).

Les mesures de fluorescence des gammes de conjugué pyrrole-$T_{10}$-k-ras et pyrrole-$T_{10}$-biotine sont illustrées par la figure 1 :

(—□— = k-ras ; —•— = biotine),

qui représente la fluorescence sur chaque électrode en fonction de la quantité initiale de conjugué présent dans la cellule lors de l'électropolymérisation, et donc du rapport molaire pyrrole/conjugué.

Cette expérience montre que le choix du rapport molaire pyrrole/P-O-M ou pyrrole/oligonucléotide permet de contrôler la quantité de molécules greffée sur chaque électrode.

**EXEMPLE 11 :**

Sur une matrice de micro-électrodes formée d'électrodes carrées de 50µm (voir exemple 6), on dépose par électro-polymérisation selon le protocole de l'exemple 6, un conjugué Pyr-$T_{10}$-Biotine, et suivant la disposition (figurée par les coordonnées i/j) indiquée sur les 2 premières lignes du tableau VI ci-dessous. 2 séries de dépôts identiques, $B_n$ et $B_n$' sont effectuées: les dépôts $B_1$ et $B_1$' sont faits successivement à l'aide de deux solutions préparées à partir de la même solution de conjugué ; on intercale un rinçage de l'électrode et de la cellule entre chaque dépôt. On procède de même avec $B_2$ et $B_2$', etc... Chaque série de dépôts est faite à l'aide de dilutions (dans l'eau) de 1/5 en 1/5, telles que [$B_2$] = [$B_1$]/5 ; [$B_3$] = [$B_2$]/5. Ces dilutions sont obtenues à partir d'une solution mère de Pyr-$T_{10}$-Biotine estimée à 125 mmol/l (mesure de $DO_{265}$ en prenant $\varepsilon_{265}$ = 120000). On utilise 8 µl de chaque dilution dans 600 µl de solution d'électro-polymérisation (pyrrole 20mM dans $LiClO_4$ 0,1 M). Les concentrations en conjugué Pyr-$T_{10}$-biotine (C), les rapports [Pyrrole]/[Pyr-$T_{10}$-biotine] (R), et les quantités de conjugué Pyr-$T_{10}$-biotine initialement présents dans la cellule d'électropolymérisation, pour chaque groupe $B_1$/$B_1$'; $B_2$/$B_2$'; etc... sont indiqués dans le tableau VI ci-dessous.

TABLEAU VI

| j / i | 1 | 2 | 3 | 4 | 5 | 6 | 7 |
|---|---|---|---|---|---|---|---|
| 1 | OR | $B_1$ | $B_2$ | $B_3$ | $B_4$ | $B_5$ | OR |
| 2 | OR | $B_1$' | $B_2$' | $B_3$' | $B_4$' | $B_5$' | OR |
| C (µmole/l) | - | 125 | 25 | 5 | 1 | 0,2 | - |
| R | - | $1,2 \times 10^4$ | $6 \times 10^4$ | $3 \times 10^5$ | $1,5 \times 10^6$ | $7,5 \times 10^6$ | - |
| Q (mol) | - | $1 \times 10^{-9}$ | $2 \times 10^{-10}$ | $4 \times 10^{-11}$ | $8 \times 10^{-12}$ | $1,6 \times 10^{-12}$ | - |

Après lavage de la plaquette d'électrodes, on révèle par incubation en présence d'un conjugué streptavidine-phycoérythrine (10 min. à 20°C, solution commerciale diluée au 1/10$^{ème}$ dans du tampon PBS 10mM pH 7,4, contenant 0,5M NaCl et 0,05% de TWEEN 20).

Après rinçage avec le même tampon, on observe la plaquette sous microscope à fluorescence, équipé d'une caméra CCD HAMAMATSU et d'un logiciel d'analyse d'image IMAGE PRO (temps d'intégration $t_i$ = 2 s ; gain × 4). A l'aide de ce logiciel, on peut mesurer point par point la fluorescence de chaque dépôt.

Les résultats sont illustrés par le Tableau VII ci-dessous, où la fluorescence pour chaque dépôt est indiquée, en unités arbitraires de fluorescence (UFA) selon le système de coordonnées (i ; j) du tableau VI, et par la Figure 2, qui représente la fluorescence en UFA en fonction de la quantité initiale de conjugué Pyr-$T_{10}$-biotine dans la cellule d'électropolymérisation (□ = série B ; • = série B').

TABLEAU VII

| j / i | 1 | 2 | 3 | 4 | 5 | 6 | 7 |
|---|---|---|---|---|---|---|---|
| 1 | 10 | 206 | 97 | 55 | 21 | 21 | 11 |
| 2 | 11 | 208 | 100 | 51 | 22 | 12 | 9 |

Ces résultats montrent clairement une relation de proportionnalité directe entre la réponse (fluorescence) et la quantité initiale de conjugué pyrrole-$T_{10}$-biotine (ou le rapport initial [pyrrole]/[pyr-$T_{10}$-biotine], la concentration en pyrrole étant constante) dans le mélange d'électropolymérisation.

L'utilisation de composée du type P-O-M permet donc de contrôler facilement la quantité de conjugué du pyrrole (pyr-$T_{10}$-bio) présent dans une solution d'électrolyte, et de réaliser des gammes d'étalonnage de matrices d'électrodes.

## Revendications

1. Macromolécule de formule (I):

$$\textbf{P-O-M} \quad \text{(I)}$$

dans laquelle :

M représente une molécule d'intérêt ;
O représente une chaîne oligonucléotidique;
P représente un monomère d'un polymère conducteur électronique.

2. Macromolécule selon la revendication 1, **caractérisée en ce que** le monomère P représente un groupe pyrrole.

3. Macromolécule selon une quelconque des revendications 1 ou 2, **caractérisée en ce que** la chaîne oligonucléotidique O est constituée d'un oligonucléotide simple-brin.

4. Macromolécule selon une quelconque des revendications 1 ou 2, **caractérisée en ce que** la chaîne oligonucléotidique O est constituée d'un oligonucléotide double-brin sur au moins une partie de sa longueur.

5. Macromolécule selon une quelconque des revendications 1 à 4, **caractérisée en ce que** la chaîne oligonucléotidique O comprend au moins 6 nucléotides, et en ce que son pourcentage en (G+C) est inférieur ou égal à 70%.

6. Procédé de préparation d'une macromolécule P-O-M selon une quelconque des revendications 1 à 4, à partir d'un mélange comprenant ladite macromolécule ainsi que les réactifs P-O et M à partir desquels elle a été formée, lequel procédé est **caractérisé en ce qu'**il comprend au moins une étape au cours de laquelle on procède au fractionnement dudit mélange, par tous moyens permettant de séparer les fractions comprenant respectivement M, et P-O-M, de la fraction comprenant P-O et une étape au cours de laquelle l'on procède à la détection de la fraction comprenant P-O-M, et/ou à la détermination de la quantité de P-O-M par détection et/ou mesure d'un paramètre associé à l'oligonucléotide O et non-associé à la molécule d'intérêt M.

7. Procédé selon la revendication 6, **caractérisé en ce que** ledit paramètre associé à l'oligonucléotide O et non associé à la molécule d'intérêt M est l'absorption en ultraviolet, à une longueur d'onde comprise entre 240 et 270 nm.

8. Copolymère, **caractérisé en ce qu'**au moins l'une de ses unités est constituée par une macromolécule selon une quelconque des revendications 1 à 5.

9. Electrode **caractérisée en ce que** sa surface porte au moins un copolymère selon la revendication 8.

10. Matrice d'électrodes, **caractérisée en ce qu'**elle comprend au moins une électrode selon la revendication 9.

11. Matrice d'électrodes selon la revendication 10, **caractérisée en ce qu'**elle comprend au moins deux électrodes différant entre elles par au moins l'un des monomères P, des oligonucléotides O et/ou des molécules M présents à leur surface.

12. Matrice d'électrodes selon la revendication 10, **caractérisée en ce qu'**elle comprend au moins deux électrodes différant entre elles par la quantité d'oligonucléotides O et/ou de molécules M par unité de surface.

13. Matrice d'électrodes selon une quelconque des revendications 10 à 12, **caractérisée en ce qu'**elle comprend en outre au moins une électrode recouverte d'un copolymère PCE/oligonucléotide.

14. Utilisation d'au moins un copolymère selon la revendication 8 pour le contrôle qualitatif et/ou quantitatif de la fixation d'au moins une molécule X sur une électrode.

15. Utilisation selon la revendication 14, **caractérisée en ce que** ladite molécule X comprend au moins l'un des constituants P, O, ou M d'une macromolécule selon la revendication 1.

16. Utilisation selon une quelconque des revendications 14 ou 15, **caractérisée en ce que** le copolymère selon la revendication 8, et la molécule X sont sur la même électrode.

17. Utilisation selon une quelconque des revendications 14 ou 15, **caractérisée en ce que** le copolymère selon la revendication 8, et la molécule X sont sur deux électrodes différentes.

18. Utilisation selon la revendication 17, **caractérisée en ce que** les 2 électrodes appartiennent à 2 matrices différentes.

## Patentansprüche

1. Makromolekül der Formel (I):

**P-O-M** (I)

worin:

M ein interessierendes Molekül darstellt;
O eine Oligonukleotidkette darstellt;
P ein Monomer eines elektronisch leitenden Polymeren darstellt.

2. Makromolekül nach Anspruch 1, **dadurch gekennzeichnet, dass** das Monomer P eine Pyrrolgruppe darstellt.

3. Makromolekül nach Anspruch 1 oder Anspruch 2, **dadurch gekennzeichnet, dass** die Oligonukleotidkette O aus einem Oligonukleotid-Einzelstrang besteht.

4. Makromolekül nach Anspruch 1 oder Anspruch 2, **dadurch gekennzeichnet, dass** die Oligonukleotidkette O über mindestens einen Teil ihrer Länge aus einem Oligonukleotid-Doppelstrang besteht.

5. Makromolekül nach einem der Ansprüche 1 bis 4, **dadurch gekennzeichnet, dass** die Oligonukleotidkette O mindestens 6 Nukleotide umfasst und dass ihr Prozentgehalt an (G+C) bei 70% oder darunter liegt.

6. Verfahren zur Herstellung eines Makromoleküls P-O-M nach einem der Ansprüche 1 bis 4, ausgehend von einem Gemisch, das das Makromolekül sowie die Reaktanten P-O und M, aus denen es gebildet wurde, umfasst, wobei das Verfahren **dadurch gekennzeichnet** ist, dass es mindestens eine Stufe, während der eine Fraktionierung des Gemisches durch beliebige Mittel, die ein Abtrennen der Fraktionen, die M bzw. P-O-M umfassen, von der Fraktion, die P-O umfasst, erlauben, durchgeführt wird, und eine Stufe, während der eine Detektion der Fraktion, die P-O-M umfasst, und/oder eine Bestimmung der P-O-M-Menge durch Detektion und/oder Messung eines Parameters, der mit dem Oligonukleotid O, nicht aber mit dem interessierenden Molekül M verbunden ist, durchgeführt wird, umfasst.

7. Verfahren nach Anspruch 6, **dadurch gekennzeichnet, dass** der Parameter, der mit dem Oligonukleotid O, nicht aber mit dem interessierenden Molekül M verbunden ist, die Absorption von ultraviolettem Licht bei einer Wellenlänge zwischen 240 und 270 nm ist.

8. Copolymer, **dadurch gekennzeichnet, dass** mindestens eine seiner Einheiten aus einem Makromolekül nach einem der Ansprüche 1 bis 5 besteht.

9. Elektrode, **dadurch gekennzeichnet, dass** ihre Oberfläche mindestens ein Copolymer nach Anspruch 8 trägt.

10. Elektrodenmatrix nach Anspruch 10, **dadurch gekennzeichnet, dass** sie mindestens eine Elektrode nach Anspruch 9 umfasst.

11. Elektrodenmatrix nach Anspruch 10, **dadurch gekennzeichnet, dass** sie mindestens zwei Elektroden umfasst, die sich durch mindestens eine der Komponenten: Monomere P, Oligonukleotide O und/oder Moleküle M, die an ihrer Oberfläche vorliegen, voneinander unterscheiden.

12. Elektrodenmatrix nach Anspruch 10, **dadurch gekennzeichnet, dass** sie mindestens zwei Elektroden umfasst, die sich durch die Menge der Oligonukleotide O und/oder der Moleküle M pro Oberflächeneinheit unterscheiden.

13. Elektrodenmatrix nach einem der Ansprüche 10 bis 12, **dadurch gekennzeichnet, dass** sie außerdem mindestens eine Elektrode umfasst, die mit einem Copolymer PCE/Oligonukleotid überzogen ist.

14. Verwendung von mindestens einem Copolymer nach Anspruch 8 zur qualitativen und/oder quantitativen Kontrolle der Fixierung mindestens eines Moleküls X auf einer Elektrode.

15. Verwendung nach Anspruch 14, **dadurch gekennzeichnet, dass** das Molekül X mindestens einen der Bestandteile P, O oder M eines Makromoleküls nach Anspruch 1 umfasst.

16. Verwendung nach Anspruch 14 oder Anspruch 15, **dadurch gekennzeichnet, dass** das Copolymer nach Anspruch 8 und das Molekül X auf derselben Elektrode sind.

**17.** Verwendung nach Anspruch 14 oder Anspruch 15, **dadurch gekennzeichnet, dass** das Copolymer nach Anspruch 8 und das Molekül X auf zwei verschiedenen Elektroden sind.

**18.** Verwendung nach Anspruch 17, **dadurch gekennzeichnet, dass** die zwei Elektroden zu zwei verschiedenen Matrices gehören.

## Claims

**1.** Macromolecule of the formula (I) :

$$\textbf{P-O-M} \qquad (I)$$

in which:

M represents a molecule of interest;
O represents an oligonucleotide chain;
P represents a monomer of an electronically conductive polymer.

**2.** Macromolecule according to Claim 1, **characterized in that** the monomer P represents a pyrrole group.

**3.** Macromolecule according to either Claim 1 or 2, **characterized in that** the oligonucleotide chain O consists of a single-stranded oligonucleotide.

**4.** Macromolecule according to either Claim 1 or 2, **characterized in that** the oligonucleotide chain O consists of an oligonucleotide which is doublestranded along at least a part of its length.

**5.** Macromolecule according to any one of Claims 1 to 4, **characterized in that** the oligonucleotide chain O comprises at least 6 nucleotides and in that its percentage of (G+C) is less than or equal to 70%.

**6.** Process for preparing a macromolecule P-O-M according to any one of Claims 1 to 4 from a mixture which comprises the said macromolecule and also the P-O and M reagents from which it was formed, which process is **characterized in that** it comprises at least one step during which the said mixture is fractionated by any means which make it possible to separate the fractions comprising M and P-O-M, respectively, from the fraction comprising P-O, and a step during which the fraction comprising P-O-M is detected and/or the quantity of P-O-M is determined by detecting and/or measuring a parameter which is associated with the oligonucleotide O and not associated with the molecule of interest M.

**7.** Process according to Claim 6, **characterized in that** the said parameter which is associated with the oligonucleotide O and not associated with the molecule of interest M is absorption in the ultraviolet at a wavelength of between 240 and 270 nm.

**8.** Copolymer, **characterized in that** at least one of its units consists of a macromolecule according to any one of Claims 1 to 5.

**9.** Electrode, **characterized in that** its surface carries at least one copolymer according to Claim 8.

**10.** Electrode matrix, **characterized in that** it comprises at least one electrode according to Claim 9.

**11.** Electrode matrix according to Claim 10, **characterized in that** it comprises at least two electrodes which differ from each other in at least one of the monomers P, the oligonucleotides O and/or the molecules M which are present on their surface.

**12.** Electrode matrix according to Claim 10, **characterized in that** it comprises at least two electrodes which differ from each other in the quantity of oligonucleotides O and/or molecules M per unit of surface area.

**13.** Electrode matrix according to any one of Claims 10 to 12, **characterized in that** it additionally comprises at least one electrode which is covered with an ECP/oligonucleotide copolymer.

**14.** Use of at least one copolymer according to Claim 8 for qualitatively and/or quantitatively monitoring the attachment of at least one molecule X to an electrode.

**15.** Use according to Claim 14, **characterized in that** the said molecule X comprises at least one of the constituents P, O or M of a macromolecule according to Claim 1.

**16.** Use according to either Claim 14 or 15, **characterized in that** the copolymer according to Claim 8 and the molecule X are on the same electrode.

**17.** Use according to either Claim 14 or 15, **characterized in that** the copolymer according to Claim 8 and the molecule X are on two different electrodes.

**18.** Use according to Claim 17, **characterized in that** the 2 electrodes belong to 2 different matrices.

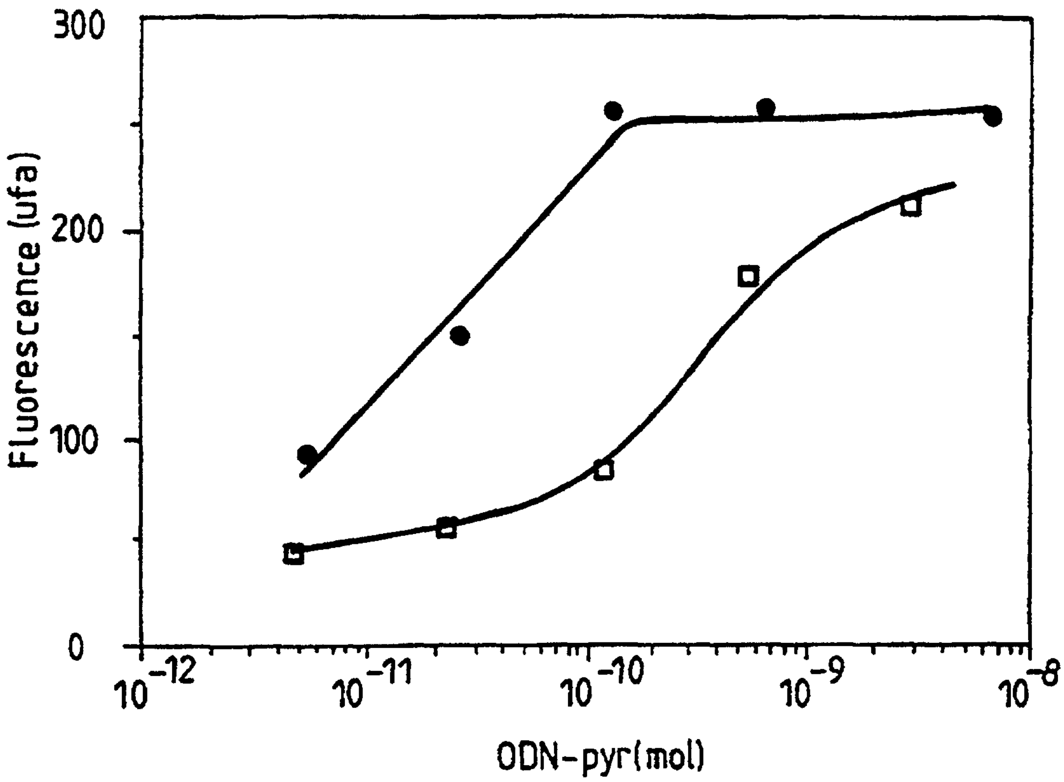
300
200
100
0
Fluorescence (ufa)
$10^{-12}$
$10^{-11}$
$10^{-10}$
$10^{-9}$
$10^{-8}$
ODN-pyr(mol)

FIG.1

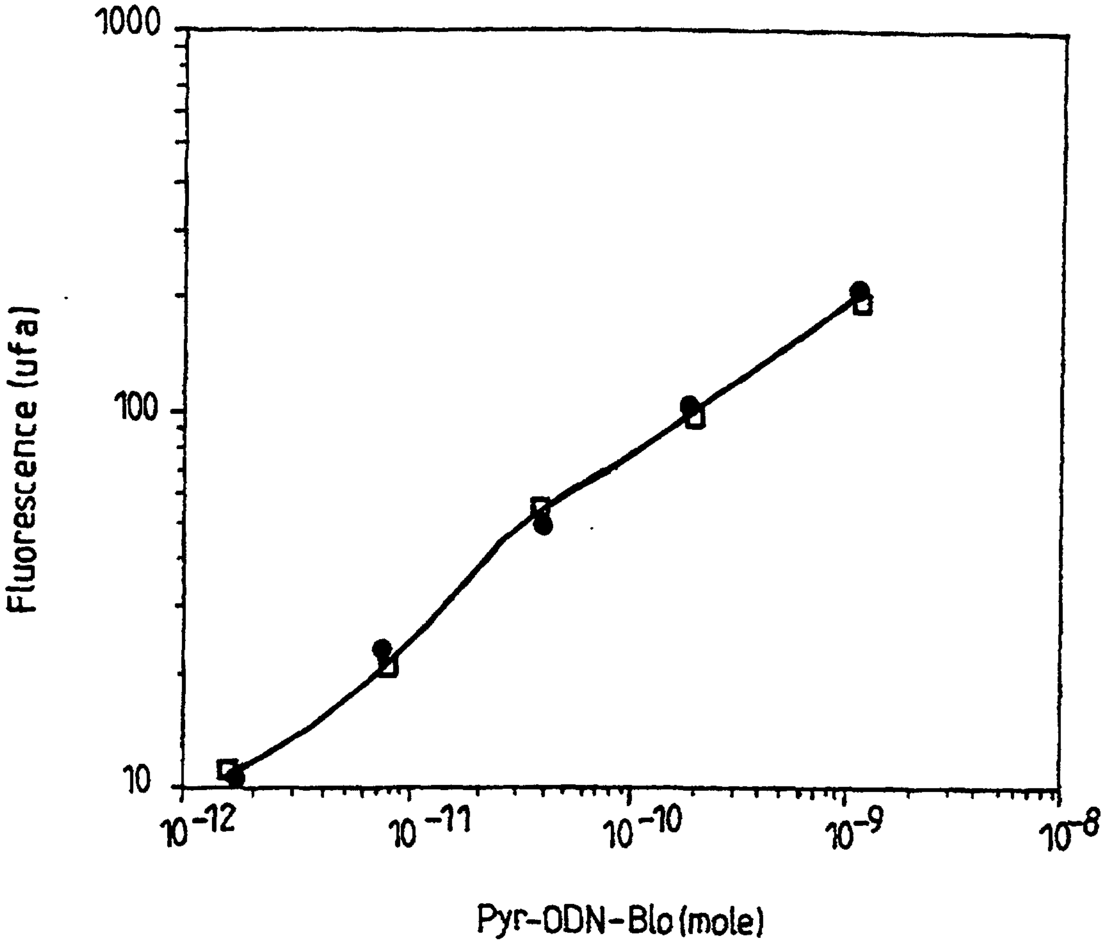
1000
100
10
Fluorescence (ufa)
$10^{-12}$
$10^{-11}$
$10^{-10}$
$10^{-9}$
$10^{-8}$
Pyr-ODN-Bio (mole)

FIG. 2